# EUROPÄISCHE PATENTSCHRIFT

(11) **EP 0 261 182 B1**
(45) Veröffentlichungstag und Bekanntmachung des Hinweises auf die Patenterteilung: **02.06.1993**
(21) Anmeldenummer: 87902050.1
(22) Anmeldetag: 20.03.1987
(51) Int. Cl.: A61F 2/46

(54) **VORRICHTUNG UND VERFAHREN ZUM UMFÜLLEN EINES VISKöSEN STOFFGEMISCHES, INSBESONDERE VON KNOCHENZEMENT**
APPARATUS AND PROCESS FOR TRANSFERRING A VISCOUS MIXTURE OF SUBSTANCES, ESPECIALLY BONE CEMENT
DISPOSITIF ET PROCEDE POUR LE TRANSVASEMENT D'UN MELANGE VISQUEUX DE SUBSTANCES, EN PARTICULIER DU CIMENT D'OS

(30) Priorität: 21.03.1986 DE 3609672
(43) Veröffentlichungstag der Anmeldung: 30.03.1988
(60) Teilanmeldung: 92102656.3
(73) Patentinhaber: DRAENERT, Klaus, Dr.med., D-81545 München (DE)
(72) Erfinder: DRAENERT, Klaus, Dr.med., D-81545 München (DE)
(74) Vertreter: VOSSIUS & PARTNER
(86) Internationale Anmeldenummer: DE8700126
(87) Internationale Veröffentlichungsnummer: WO8705492

(56) Entgegenhaltungen:
- EP-A- 0 170 120
- DE-A- 3 347 843
- US-A- 3 779 371

## Beschreibung

Die Erfindung betrifft eine Vorrichtung und ein Verfahren zum Umfällen eines aus mindestens zwei Bestandteilen bestehenden viskösen Stoffgemisches. Insbesondere betrifft die Erfindung eine Vorrichtung und ein Verfahren zum Umfüllen von Knochenzement von einem Mischbecher in einen Applikationsbehälter.

Unter Mischen versteht man allgemein das Einbringen von Teilen eines Stoffes zwischen die Teile anderer Stoffe. Ziel ist dabei eine möglichst homogene Verteilung der verschiedenen Bestandteile des Stoffgemisches, um beispielsweise eine chemische Reaktion zwischen den Bestandteilen einzuleiten oder zu fördern, z. B. die nachfolgende Polymerisation eines Zwei- oder Mehrkomponentengemisches.

Es sind verschiedene Arten von Mischverfahren und -vorrichtungen bekannt. Das Mischen erfolgt dabei beispielsweise durch Rühren, Mengen, Walzen, Kneten, Emulgieren, Suspendieren, Lösen oder durch Einwirkung von Ultraschall.

Die bekannten Mischverfahren und -vorrichtungen weisen jedoch den Nachteil auf, daß Verunreinigungen, beispielsweise Luft, in das Mischsystem eingetragen werden können und daß die bereits zu Beginn des Mischvorganges in den zu mischenden Stoffen enthaltenen und die während des Mischvorganges, beispielsweise durch chemische Reaktionen entstehenden Gaseinschlüsse nicht aus dem Mischsystem entfernt werden können. Ferner besteht ein Nachteil häufig darin, daß der Mischbehälter nicht mit dem Behälter identisch ist, in dem die weitere Verarbeitung des durchmischten Stoffgemisches erfolgen soll. Es ist dann ein Transport des Stoffgemisches erforderlich, der insbesondere bei im Stoffgemisch ablaufenden Reaktionen, beispielsweise einer Polymerisation, Probleme bereitet.

Spezielle Probleme treten beim Verarbeiten und Mischen von Knochenzement und beim Einfüllen oder Umfüllen in den Behälter auf, aus dem der Knochenzement appliziert wird.

Als Knochenzement werden meist kalt polymerisierende Zweikomponenten-Kunststoffe verwendet, durch die die Komponenten künstlicher Gelenke im knöchernen Bett verankert werden. Der Knochenzement härtet unmittelbar nach der Applikation aus und erreicht durch seine plastischen Eigenschaften eine Verblockung der Prothesenkomponente im knöchernen Lager. Seit vielen Jahren werden als Knochenzemente Polymethylmethacrylate (PMMA) verwendet. Diese bestehen aus einem pulverförmigen Perlpolymerisat, welches in flüssigem Monomer angelöst und schließlich durch Polymerisation des Monomers in dieses eingebettet wird. In der Mischphase umfließt das Monomer das etwa kugelförmige Polymerpulver. Dabei gibt es zunächst eine Kugelaufschwemmung, in der mehr oder weniger viele Luftblasen eingeschlossen sind. Die Polymerisation läuft exotherm ab. Neben den eingeschlossenen Luftblasen kommt es regelmäßig beim Umfließen der Polymerkugeln durch das Monomer zu sogenannten "Windschattenphänomenen", also einer unzureichenden Benetzung der Polymerkugeln, und während der exothermen Polymerisation auch zum Verdampfen der monomeren Flüssigkeit, so daß letztlich der ausgehärtete Knochenzement von Blasen unterschiedlicher Ethiologie und Genese durchsetzt ist.

In der Regel wird das Polymerpulver dem Monomer beigegeben und mit einem Spatel in einer Schale vermischt. In der an die Mischphase anschließenden Verarbeitungsphase wird der Knochenzement meist von Hand, teilweise auch mit einer Spritze, in das knöcherne Lager eingebracht, beispielsweise in die Femurmarkhöhle oder in das knöcherne Acetabulum, die für die Verankerung der zementierten Prothesenkomponenten vorbereitet wurden. Eine derartige Spritze ist beispielsweise in der DE-A-28 01 706 oder in der EP-A1-170 120 beschrieben. Durch Verwendung einer Knochenzementspritze konnten im Hinblick auf die Zementverankerung im Knochen wesentlich bessere Ergebnisse als mit der herkömmlichen Fingerstopfmethode erzielt werden.

Es gibt bisher kaum Arbeiten, die sich mit der Misch- oder Quellphase des Knochenzementes und dem artefaktfreien Einfüllen oder Umfüllen in das Spritzensystem befassen.

Die weitere Verarbeitung des in der vorstehend erläuterten Weise in der Mischschale angerührten Knochenzementes hängt von dessen Viskosität ab. Sehr niedrigvisköse Knochenzemente können aus der Schale in die Kartusche der Knochenzementspritze gegossen werden; hierbei tritt jedoch das Problem auf, daß der einfließende Knochenzementstrahl sehr leicht, z. B. durch elektrostatische Aufladung abgelenkt wird, so daß fast immer die Wand der Kartusche und die Einfüllöffnung mit Knochenzement verschmiert werden. Hochvisköse Zemente lassen sich überhaupt nicht gießen. Sie müssen von Hand herausgenommen und geknetet werden, damit die gröbsten Lufteinschlüsse herausgedrückt werden. Anschließend wird der Knochenzement zu einer wurstförmigen Masse gerollt, die dann in die Kartusche eingeführt werden kann. Die Handverarbeitung des Zementes erfordert nicht nur ein Warten, bis der Knochenzement nicht mehr an den Operationshandschuhen kleben bleibt, sondern läßt die Zementmischung auch in der wertvollsten Zeit der Quellphase und der sich daran anschließenden Vorpolymerisationsphase unbearbeitet.

Die bisher vorgeschlagenen Versuche, das Problem des Zementmischens in einem sogenannten "geschlossenen System" in den Griff zu bekommen, führten zu keinen besseren Mischungen als die von Hand ausgeführten Ansätze.

Der Erfindung liegt somit die Aufgabe zugrunde, ein Verfahren und eine Vorrichtung bereitzustellen, mit denen ein aus mindestens zwei Bestandteilen bestehendes visköses Stoffgemisch rasch und blasenfrei durchmischt und in den Behälter umgefüllt werden kann, in dem die weitere Verarbeitung des Stoffgemisches stattfinden soll.

Eine weitere Aufgabe der Erfindung besteht darin, eine Vorrichtung und ein Verfahren zum Mischen und Umfüllen von Knochenzement in einen Applikationsbehälter bereitzustellen, mit denen der aus mehreren Komponenten bestehende Knochenzement rasch, blasenfrei und ohne Berührung durch den Operateur durchmischt und in den Applikationsbehälter umgefüllt werden kann, aus dem heraus er in das knöcherne Lager appliziert werden soll.

Diese Aufgaben werden durch die Merkmale der Patentansprüche gelöst.

Die Erfindung geht dabei von dem Grundgedanken aus, die zu mischenden Stoffe, vorzugsweise ein aushärtbares Zweikomponentensystem, also beispielsweise das Gemisch aus Polymerpulver und Monomer, von einem ersten Behälter durch eine Öffnung oder Verjüngung in einen zweiten Behälter hineinzudrücken und dadurch umzufüllen. Dieser Vorgang läßt sich als "Extrusionsmischen" bezeichnen.

Vorzugsweise ist der erste Behälter oder Mischbehälter becherförmig ausgebildet bzw. zylindrisch mit einem geschlossenen und einem offenen Ende. Der zweite Behälter, der gleichzeitig als Applikationsbehälter dient, in dem das Stoffgemisch nach der Durchmischung zur Weiterverarbeitung verbleibt, ist vorzugsweise ebenfalls zylindrisch ausgebildet mit einem offenen Ende. Das andere Ende des zweiten Behälters ist durch eine Kappe oder einen Stempel verschließbar. Der zweite Behälter trägt an seinem Außenumfang, vorzugsweise an seinem offenen Ende, eine Abdichteinrichtung, die vorzugsweise aus mehreren flexiblen Scheiben oder Lamellen besteht.

Zur Mischung und Umfüllung des viskösen Stoffgemisches wird der zweite zylindrische Behälter oder Hohlkörper mit seinem offenen Ende voran in Axialrichtung in den ersten Behälter hineingedrückt, in dem sich die zu mischenden oder bereits teilweise gemischten Stoffe befinden. Durch diese Relativbewegung der beiden Behälter wird das Stoffgemisch durch die Öffnung in den zweiten Behälter hineingedrückt, da die Abdichteinrichtung den Zwischenraum zwischen der Innenwand des ersten Behälters und der Außenwand des zweiten Behälters derart abschließt, daß lediglich Gas, aber nicht die zu mischenden Stoffe passieren können.

Bei der Anwendung des vorstehenden Prinzips auf das Mischen und Umfüllen von Knochenzement in einen Applikationsbehälter bzw. eine Kartusche wird zunächst das Monomer in einem Mischbecher vorgelegt und danach das Polymerpulver in den Ansatz gebracht und mit einem Spatel aus Metall, Kunststoff oder Holz untermischt und verrührt. Anstatt nun wie bei herkömmlichen Verfahren den gesamten Zementbrei mit dem Spatel herauszunehmen oder bei niedrigviskösen Zementen herauszugießen, wird erfindungsgemäß von der Öffnung des Mischbechers her der als Kartusche ausgebildete Applikationsbehälter mit der aufgesetzten Abdichteinrichtung eingesetzt. Wenn die Abdichteinrichtung als zylinderförmiger Körper mit zentraler, rohrförmiger Öffnung und mehreren scheibenförmigen Lamellen ausgebildet ist, kann die Abdichteinrichtung unter Einsetzen von Adaptern, vorzugsweise kleinen Ringen, auf alle handelsüblichen Kartuschen aufgesetzt und zusammen mit diesen verwendet werden. Vorzugsweise wird der in der EP-A1-170 120 erläuterte zylinderförmige Behälter als Applikationsbehälter bzw. Kartusche verwendet. Durch Eindrücken der Kartusche mit der damit gekoppelten Abdichteinrichtung wird der durch den Spatel vorgemischte Zement durch die zentrale Öffnung der Abdichteinrichtung in die Kartusche hineingedrückt. Die Öffnung kann dabei entweder den gleichen oder kleineren Durchmesser als die Kartusche aufweisen. Durch die Extrusionswirkung findet eine weitere Durchmischung des Knochenzementes statt und der Knochenzement gelangt ohne Berührung durch den Operateur vom Mischbecher in die Kartusche, wobei durch den Fließvorgang zusätzlich größere Luftblasen herausgedrückt werden.

Zum Vormischen des Zements wird anstelle herkömmlicher Spatel vorzugsweise ein Rundstab verwendet, der vorteilhafterweise mit Teflon beschichtet ist. Der Rundstab hat den Vorteil, daß er das Gemisch nicht zerreißt, sondern dessen Durchmischung durch eine laminare Strömung der Schichten des Gemisches fördert. Außerdem bleibt am Teflonrundstab beim Rühren und Herausziehen praktisch kein Zement haften.

Besonders vorteilhaft zur Vermeidung von Lufteinschlüssen ist es, das Mischen der Bestandteile, beispielsweise der Komponenten des Knochenzementes, unter Vakuum durchzuführen. Hierzu weist der Mischbehälter vorzugsweise einen plangeschliffenen oberen Rand auf, auf den ein Deckel aufgesetzt wird. Die Abdichtung zwischen dem Mischbehälter und dem Deckel kann durch einen Dichtring mit Vakuumfett oder eine Silikonbeschichtung erfolgen. Der Deckel kann auch durch eine rasch lösbare Flanschverbindung mit dem Mischbehälter vakuumdicht verbunden werden. Der Deckel weist einen Anschluß für eine Schlauchzuführung auf, an die eine Vakuumpumpe angeschlossen wird.

Vorzugsweise weist der Deckel einen festen Rand zur Auflage auf dem Rand des Mischbehälters und eine Durchführung für den zum Rühren verwendeten Rundstab auf. Die abgedichtete Durchführung für den Rundstab ist vorzugsweise innerhalb eines aus flexiblem Material bestehenden Innenteils des Deckels angeordnet, der mit dem festen Rand vakuumdicht, vorzugsweise einstückig verbunden ist. Das Innenteil des Deckels kann zeltförmig ausgebildet sein und an seinem oberen Ende die als eine ringförmige Führung ausgebildete Durchführung, vorzugsweise aus Gummi oder Silikon aufweisen, durch die der Rundstab geführt wird und die den Rundstab vakuumdicht festhält. Durch den flexiblen Innenteil des Deckels ist der Rundstab in Radialrichtung im Mischbehälter beweglich und kann an der Innenwand des Mischbehälters entlang geführt werden, so daß kein Teil der zu mischenden Bestandteile, beispielsweise kein Pulver des Knochenzements, unberührt an der Wand liegen bleibt. Dies ist für eine gute Durchmischung außerordentlich wichtig.

Durch die Mischung unter Vakuum können die Blasen im Zement noch weiter reduziert und das Gemisch praktisch blasenfrei gerührt werden.

Bei Versuchen hat sich herausgestellt, daß hohe Mischbecher zu einer sehr viel schnelleren und homogeneren Mischung der Zementmasse führen als weite, flache und an den Ecken totrandige Mischschalen. Vorzugsweise ist der Boden des Mischbechers innen sphärisch oder konkav ausgebildet. Beim Hineindrücken der Kartusche paßt sich die vorderste, flexible Lamelle der Abdichteinrichtung dem Innenboden des Mischbechers abdruckmäßig derart an, daß keine Reste im Mischbecher zurückbleiben. Besonders vorteilhaft ist es hierbei, wenn der Innenboden des Mischbechers ebenfalls flexibel ist, so daß eine vollständige Anpassung der Form gewährleistet ist.

Toträume während des Fließvorganges am oberen Rand der Zementmasse können dadurch verhindert werden, daß die vorderste Lamelle der Abdichteinrichtung ebenfalls etwas gekrümmt bzw. konkav ist, so daß die Zementmasse vom Außenrand des Mischbechers radial nach innen gedrückt wird.

Wie vorstehend erläutert, können während des Hineindrückens der Kartusche mit der Abdichteinrichtung Luft und die während der Polymerisation frei werdenden Gase an den Lamellen der Abdichteinrichtung vorbei entweichen. Der Körper der Abdichteinrichtung kann auch mindestens ein zusätzliches Ventil aufweisen, durch das die Gase ausweichen können.

Ferner kann auch in der Mischphase durch Anwendung von Druck und Verschließen der zentralen Öffnung oder des Kartuschenendes bei gleichzeitiger mechanischer Komprimierung des Zementes ein Vakuum angelegt und Luft aus dem Mischbecher abgesaugt werden, so daß auch kleine Luftblasen bereits während des Mischvorganges zum größten Teil aus dem Zement entfernt werden können.

Beim Einfüllen des Zementes in die Kartusche ist es wichtig, daß diese an ihrem vom Mischbecher abgewandten Ende nicht verschlossen ist oder, falls sie eine Kappe aufweist, die Kappe nur locker aufgesetzt ist, damit die vor dem Zement herausgedrückte Luft aus der Kartusche entweichen kann.

Bei dem vorstehend erläuterten System, das als "halbgeschlossenes System" bezeichnet werden kann, kann als zweiter Behälter unmittelbar eine Kartusche verwendet werden, wie sie in einer Knochenzementspritze gemäß der EP-A1-170 120 oder in ähnlichen Spritzen verwendet wird. Bei diesem Sysem muß der Zement bis zur Applikation überhaupt nicht mehr mit den Händen und Operationshandschuhen des Operateurs oder der Operationsschwester in Berührung kommen, wodurch große Vorteile erzielt werden. Zum einen ist es bekannt, daß das Monomer die Gummihandschuhe des Operationspersonals leicht durchdringen kann, und es wurden in letzter Zeit immer mehr Allergien gegen den Kunststoff bekannt. Zum anderen können durch das berührungsfreie Vorgehen auch die Fehleinschlüsse im Zement erheblich verringert werden und der Zement gelangt rascher und früher zur Vorkomprimierung in die Knochenzementpistole, weil das Abbinden des Zementes nicht abgewartet werden muß.

Vorzugsweise bestehen der erste Behälter ( Mischbecher) und der zweite Behälter ( Applikationsbehälter) aus demselben Material. Es kann ein Kunststoff, vorzugsweise ein thermoplastischer Kunststoff, wie ein Polyolefin verwendet werden. Besonders bevorzugt ist die Verwendung von Poly( 4-methyl-1-penten) oder TPX®. Es kann auch Polycarbonat verwendet werden.

Der Vakuumdeckel für den Mischbehälter besteht vorzugsweise ebenfalls aus Teflon oder dem für den Mischbehälter und den Applikationsbehälter verwendeten Material, beispielsweise Poly( 4-methyl-1-penten).

Die Erfindung wird nachstehend anhand von Ausführungsbeispielen, die das Mischen und Umfüllen von Knochenzement in einen Applikationsbehälter betreffen, und anhand der Zeichnung näher erläutert. Es zeigen:
Fig. 1 eine Ausführungsform einer erfindungsgemäßen Vorrichtung zum Mischen und Umfüllen von Knochenzement in einen Applikationsbehälter, und
Fig. 2 eine ähnliche Ausführungsform wie Fig. 1 mit einem zusätzlichen Vakuumdeckel für den Mischbehälter.

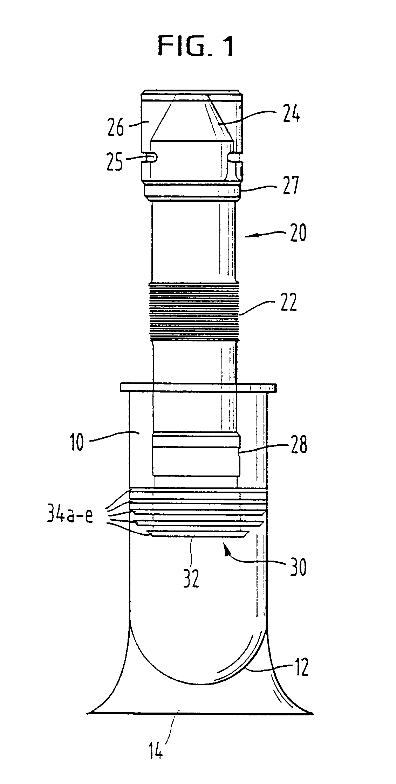

Gemäß Fig. 1 weist die erfindungsgemäße Vorrichtung einen hohen, kreiszylindrischen Mischbecher 10 mit einem runden inneren Boden 12 und einer ebenen Standfläche 14 auf.

Der als Spritzenkartusche ausgebildete Applikationsbehälter 20 weist eine geriffelte Griffläche 22 und ein als Mundstück für die Applikation dienendes konisches vorderes Ende 24 auf. An diesem Ende ist mittels eines Bajonettverschlusses 25 mit steigendem Gewinde eine Verschlußkappe 26 befestigt, die an einer als Anschlag dienenden Rippe 27 am Applikationsbehälter 20 anliegt. Am anderen, bei der Applikation hinteren Ende des Behälters 20 ist mittels eines Adapters 28 eine Abdichteinrichtung 30 befestigt. Der rohrförmige Körper 32 der Abdichteinrichtung 30 weist in seinem Inneren eine zentrale Öffnung und an seinem Umfang fünf flexible Lamellen 34 a - e auf. Die Abdichteinrichtung 30 ist aus Teflon hergestellt und der Durchmesser der Lamellen nimmt von der dem Boden des Mischbechers 10 zugewandten vordersten Lamelle 34 e, die den kleinsten Durchmesser aufweist, allmählich zu.

Beim Mischen des Knochenzements wird zunächst das Monomer und danach das Polymerpulver in den Mischbecher 10 gegeben und mit einem Spatel, vorzugsweise einem Rundspatel aus Teflon gemischt. Danach wird der Applikationsbehälter 20 mit der aufgesetzten Abdichteinrichtung 30, wie in Fig. 1 dargestellt, von oben in den oben offenen Mischbecher 10 hineingedrückt. Hierbei passen sich die Lamellen an den runden inneren Boden 12 des Mischbechers 10 an, und der Knochenzement wird vollständig durch die zentrale Öffnung der Abdichteinrichtung 30 in den Behälter 20 hineingepreßt. Die Lamellen 34 sind derart ausgebildet, daß zwar den Knochenzement überstehende Luft und/ oder aus dem Knochenzement bei der Polymerisation entweichende Gase an den Lamellen 34 vorbei streichen können, daß jedoch der Knochenzement höchstens einen Teil der Lamellen passieren kann. Durch die abgestuften Durchmesser der Lamellen 34 ist sichergestellt, daß auch bei gewissen Fertigungstoleranzen der Knochenzement nicht alle Lamellen passieren kann.

Da das Rohgemisch aus Polymerpulver und Monomer nicht die Abdichteinrichtung 30 passieren kann, wird durch die Kompression das Rohgemisch aus dem Mischbecher 10 in den Applikationsbehälter 20 extrudiert. Durch eine konkave Form der vordersten Lamelle werden Toträume an der Wand des Mischbechers besonders gut vermieden und das Rohgemisch radial nach innen zur Öffnung des Applikationsbehälters gedrückt, ohne daß Reste an der Wand zurückbleiben. Wenn der Applikationsbehälter bis zum Boden des Mischbechers gedrückt ist und sich die flexible vorderste Lamelle vollständig an die Form des inneren Bodens des Mischbechers anpaßt, befindet sich das Knochenzementgemisch vollständig im Applikationsbehälter.

Nachdem auf diese Weise der gesamte Knochenzement in den Behälter 20 eingefüllt ist, wird der Adapter 28 mit der Abdichteinrichtung 30 vom Behälter 20 gelöst und der Behälter 20 wird auf eine Knochenzementspritze, beispielsweise die Knochenzementpistole gemäß der EP-A1-170 120 aufgesetzt. Der Applikationsbehälter 20 dient somit unmittelbar als Kartusche zum Applizieren des Knochenzements.

Das gegebenenfalls mittels eines Adapters aufgesetzte Mundstück 24 am Vorderende des Applikationsbehälters 20 kann je nach Anwendung für Pfanne, Femur oder Kniegelenk verschieden sein.

Seitlich am Umfang kann der Mischbecher eine geriffelte Griffläche aufweisen, und der innere Boden des Mischbechers kann konkav ausgebildet sein. Der Applikationsbehälter kann eine umlaufende Rippe aufweisen. Die Abdichteinrichtung kann auch drei scheibenförmige, flexible Lamellen aufweisen. Die vorderste Lamelle kann eine konkave, sich in Richtung auf den Boden des Mischbechers öffnende Flächebilden. Auch die anderen Lamellen können konkav sein. Im Körper der Abdichteinrichtung kann ein nach oben gasdurchlässiges Nadelventil vorgesehen sein.

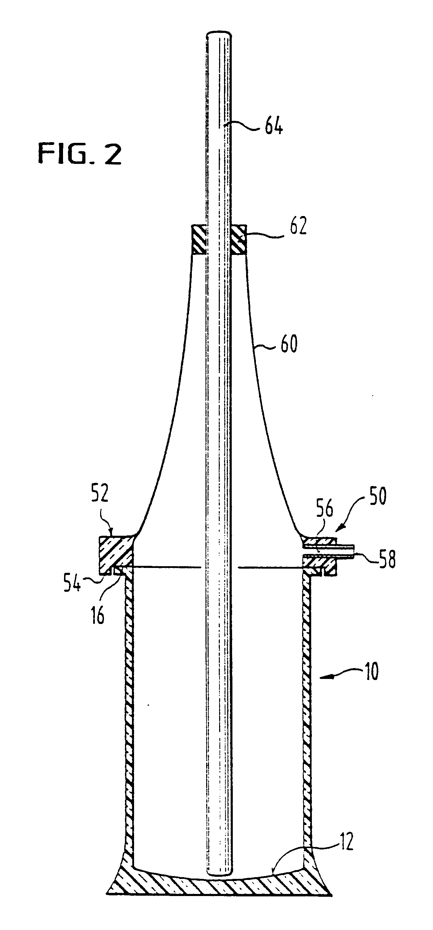

Der Mischbecher 10 gemäß Fig. 2 entspricht im wesentlichen dem Mischbecher gemäß Fig. 1 und weist einen konkaven Innenboden 12 und einen plangeschliffenen oberen Rand 16 auf. Zusätzlich zum Mischbecher gemäß Fig. 1 weist der Mischbecher 10 gemäß Fig. 2 einen Vakuumdeckel 50 auf, dessen fester Rand 52 auf dem plangeschliffenen Rand 16 des Mischbechers 10 aufliegt. Zur Abdichtung zwischen den beiden in Kontakt stehenden Flächen der Ränder 16 und 52 kann eine Silikonschicht verwendet werden, es kann aber auch ein Dichtring in einer Nut des Randes 16 vorgesehen sein. Der Rand 52 des Deckels 50 weist außen eine Nase 54 auf, die den Rand 16 übergreift und ein Verschieben des Deckels 50 gegenüber dem Rand 16 des Mischbechers 10 verhindert.

Ferner ist im Rand 52 des Deckels 50 eine Durchführung 56 und ein Stutzen 58 zum Anschluß einer ( nicht dargestellten) Vakuumleitung vorgesehen, die zu einer ebenfalls nicht dargestellten Pumpe führt.

Neben dem festen Rand 52 weist der Deckel 50 ein flexibles, zeltförmiges Innenteil 60 aus. In der dargestellten Ausführungsform ist das Innenteil 60 einstückig mit dem Rand 52 verbunden und besteht aus demselben Material wie der Rand 52, ist allerdings als dessen Fortsetzung dünner ausgezogen, so daß das Material des Innenteils 60 eine gewisse Flexibilität aufweist. Als Material hierfür eignet sich insbesondere Teflon oder Poly( 4-methyl-1-penten). Das flexible Innenteil 60 kann aber auch durch eine vakuumdichte Verbindung, beispielsweise eine Flanschverbindung, mit dem Deckel 52 verbunden sein und aus einem anderen Material als der Deckel 52 bestehen, beispielsweise aus einer Kunststoffolie.

Am oberen Ende des dach- oder zeltförmigen Innenteils 60 ist eine ringförmige Durchführung 62 aus einem dehnbaren Material, vorzugsweise einem sterilen Gummi oder Silikon, vakuumdicht eingeschweißt. Das Innenteil 60 mit der Durchführung 62 kann vorgespannt sein, so daß es eine gewisse Stabilität aufweist. Durch die flexible Durchführung 62 ist ein Rundstab 64 geführt, der vorzugsweise aus Teflon besteht und etwa 8 mm dick ist.

Die Vorrichtung gemäß Fig. 2 wird zum Rühren oder Vormischen des Rohgemisches aus Monomer und Polymerpulver im Mischbecher 10 unter Vakuum etwa in den ersten 30 Sekunden der Mischphase verwendet. Durch die flexible Ausbildung des Innenteils 60 des Deckels 50 ist der Rundstab 64 innerhalb des Mischbechers 10 in Radialrichtung beweglich und kann auch an der Innenwand des Mischbechers 10 entlang geführt werden. Hierbei ist wichtig, daß der Deckel 50 nach innen nicht gegenüber der Innenwand des Mischbechers 10 vorspringt. Der Rundstab wird verwendet, um ein Haften des Zements beim Rühren und späteren Herausziehen des Stabes zu verhindern und das Gemisch nicht zu zerreißen, sondern eine laminare Strömung der einzelnen Schichten des Gemisches zu erzeugen, durch die die Durchmischung gefördert wird.

Nach dem Abschluß der Rühr- und Mischphase wird der Rundstab 64 herausgezogen und der Deckel 50 vom Mischbecher 10 gelöst. Anschließend kann der Knochenzement, wie anhand von Fig. 1 erläutert, in den Applikationsbehälter extrudiert werden.

Die Erfindung ist nicht auf die vorstehenden Ausführungsbeispiele beschränkt, die das Mischen und Einfüllen von Knochenzement in einen Applikationsbehälter betreffen, sondern kann allgemein zum Mischen eines aus mindestens zwei Bestandteilen bestehenden Stoffgemisches und Umfüllen des durchmischten Stoffgemisches in einen Behälter verwendet werden. Die vorstehend beschriebenen Ausführungsbeispiele der erfindungsgemäßen Vorrichtung sind daher allgemein zum Mischen eines aus mindestens zwei Bestandteilen bestehenden Stoffgemisches und Einfüllen des Stoffgemisches in einen Behälter anwendbar.

Bei den vorstehend beschriebenen Ausführungsbeispielen wird die zum Mischen und Einfüllen des Gemisches erforderliche Relativbewegung des ersten Behälters und des zweiten Behälters durch Betätigung per Hand erzielt. Selbstverständlich kann das erfindungsgemäße Misch- und Umfüllprinzip, insbesondere auch bei bezüglich des Mischvolumens größeren Systemen, auch durch maschinelle Betätigung der einzelnen Teile realisiert werden. Hierfür können gegebenenfalls lösbar in Eingriff bringbare Antriebeinrichtungen vorgesehen sein.

## Patentansprüche

1. Vorrichtung zum Umfüllen eines aus mindestens zwei Bestandteilen bestehenden viskösen Stoffgemisches, mit
a) einem ersten, an einem Ende geschlossenen Behälter (10),
b) einem zweiten Behälter (20), dessen Querschnitt kleiner ist als der des ersten Behälters (10), dessen dem ersten Behälter zugewandtes Ende eine Öffnung aufweist, dessen dem ersten Behälter abgewandtes Ende gasdurchlässig ist, und der im ersten Behalter (10) und relativ zu diesem beweglich ist, und
c) einer am äußeren Umfang des zweiten Behälters (20) in der Nähe von dessen offenem Ende angeordneten Abdichteinrichtung (30), die bei einer durch Hineindrücken des zweiten Behälters (20) mit seinem offenen Ende voran in den ersten Behälter (10) durchgeführten Relativbewegung der beiden Behälter zueinander den Zwischenraum zwischen dem inneren Umfang des ersten Behälters (10) und dem äußeren Umfang des zweiten Behälters (20) für das Stoffgemisch und dessen Bestandteile abdichtet, und die eine für den Durchtritt des viskösen Stoffgemisches freie Öffnung zum Extrudieren des Stoffgemisches vom ersten Behälter (10) in den zweiten Behälter (20) aufweist.

2. Vorrichtung nach Anspruch 1, dadurch gekennzeichnet, daß das visköse Stoffgemisch Knochenzement ist, der erste Behälter (10) ein Mischbecher mit einem Boden (12) ist und der zweite Behälter (20) ein Applikationsbehälter ist.

3. Vorrichtung zum Umfüllen von Knochenzement mit
a) einem Mischbecher (10) mit einem Boden (12),
b) einem Applikationsbehälter (20), dessen Querschnitt kleiner ist als derjenige des Mischbechers (10), und der in dem Mischbecher (10) und relativ zu diesem beweglich ist, wobei das dem Boden (12) des Mischbechers (10) zugewandte Ende des Applikationsbehälters (20) eine Öffnung zum Durchtritt des Knochenzements aufweist und das dem Boden (12) des Mischbechers (10) abgewandte Ende des Applikationsbehälters (20) gasdurchlässig ist, und
c) einer mit dem Applikationsbehälter (20) koppelbaren Abdichteinrichtung (30) zum zementdichten Abdichten zwischen dem Außenumfang des Applikationsbehälters (20) und dem Innenumfang des Mischbechers (10).

4. Vorrichtung nach Anspruch 2 oder 3, gekennzeichnet durch einen Deckel (50) zum vakuumdichten Verschließen des Mischbechers (10) mit einem Stutzen (58) zum Anschließen einer Vakuumleitung.

5. Vorrichtung nach Anspruch 4, dadurch gekennzeichnet, daß der Deckel (50) einen festen Rand (52) und ein aus flexiblem Material bestehendes Innenteil (60) mit einer Durchführung (62) für einen Rundstab (64) aufweist.

6. Vorrichtung nach einem der Ansprüche 2 bis 5, dadurch gekennzeichnet, daß der Mischbecher (10) zylindrisch ist und sein Boden (12) innen konkav oder rund ausgebildet ist.

7. Vorrichtung nach einem der Ansprüche 1 bis 6, dadurch gekennzeichnet, daß die Abdichteinrichtung (30) mindestens ein Ventil zum Gasdurchlaß aufweist.

8. Vorrichtung nach einem der Ansprüche 1 bis 7, dadurch gekennzeichnet, daß die Abdichteinrichtung (30) einen zylinderförmigen Körper (32) mit mehreren flexiblen Lamellen (34) an seinem Außenumfang aufweist, die am Innenumfang des Mischbechers (10) anliegen.

9. Vorrichtung nach Anspruch 8, dadurch gekennzeichnet, daß mindestens die vorderste, dem Boden des Mischbechers zugewandte Lamelle konkav ist.

10. Vorrichtung nach einem der Ansprüche 2 bis 9, gekennzeichnet durch ein mit einem Ende des Applikationsbehälters (20) verbundenes oder koppelbares Mundstück (24).

11. Vorrichtung nach einem der Ansprüche 2 bis 10, gekennzeichnet durch eine Einrichtung zum Evakuieren des Mischbechers ( 10) und/oder des Applikationsbehälters (20).

12. Verfahren zum Umfüllen von Knochenzement mit einer Vorrichtung nach einem der Ansprüche 2 bis 11, dadurch gekennzeichnet, daß flüssiges Monomer und Polymerpulver in dem Mischbecher (10) zusammengebracht und gemischt werden und das Knochenzement gemisch anschließend durch Kompression durch die Öffnung in den Applikationsbehälter (20) extrudiert wird.

## Claims

1. An apparatus for transferring a viscous mixture of substances consisting of at least two components, comprising
a) a first vessel (10), closed at one end,
b) a second vessel (20), the cross-section of which is smaller than that of the first vessel (10), wherein the end of the second vessel which faces the first vessel has an opening and the end which faces away from the first vessel is gas permeable, and which can be moved within the first vessel (10) and relative thereto, and
c) a sealing means (30) at the outer circumference of the second vessel (20) near the open end thereof, which seals off the space between the inner circumference of the first vessel (10) and the outer circumference of the second vessel (20) for the mixture of substances and the components thereof when the two vessels move relative to each other as a result of pushing the second vessel (20) with its open end first into the first vessel (10), and which comprises an opening for extruding the mixture of substances from the first vessel (10) into the second vessel (20), which opening is free for the penetration of the viscous mixture of substances.

2. The apparatus according to claim 1, characterized in that the viscous mixture of substances is bone cement, the first vessel (10) is a mixing bowl with a bottom (12) and the second vessel (20) is an applicator.

3. An apparatus for transferring bone cement, comprising
a) a mixing bowl (10) with a bottom (12),
b) an applicator (20), the cross-section of which is smaller than that of the mixing bowl (10), and which can be moved in the mixing bowl and relative thereto, wherein the end of the applicator (20) which is facing the bottom of the mixing bowl (10) comprises an opening for the penetration of the bone cement and wherein the end of the applicator (20) which is facing away from the bottom (12) of the mixing bowl (10) is gas permeable, and
c) a sealing means (30), which is connectable with the applicator (20), for sealing between the outer circumference of the applicator (20) and the inner circumference of the mixing bowl (10) in a cement-tight manner.

4. The apparatus according to claim 2 or 3, characterized by a lid (50) for sealing the mixing bowl (10) in a vacuum-tight manner, said lid comprising a connection piece (58) for connection with a vacuum line.

5. The apparatus according to claim 4, characterized in that the lid (50) has a firm rim (52) and an inner portion (60) made of flexible material, said inner portion comprising a feed-through (62) for a round rod (64).

6. The apparatus according to any one of claims 2 to 5, characterized in that the mixing bowl (10) is cylindrical and the interior of the bottom thereof is concave or round.

7. The apparatus according to any one of claims 1 to 6, characterized in that the sealing means (30) comprises at least one valve for gas passage.

8. An apparatus according to any one of claims 1 to 7, characterized in that the sealing means (30) comprises a cylindrical body (32) having several flexible lamellae (34) on its outer circumference, which contact the inner circumference of the mixing bowl (10).

9. The apparatus according to claim 8, characterized in that at least the foremost lamella which faces the bottom of the mixing bowl (10) is concave.

10. The apparatus according to any one of claims 2 to 9, characterized by a mouth piece (24) which is connected to or can be coupled with one end of the applicator (20).

11. The apparatus according to any one of claims 2 to 10, characterized by a means for evacuating the mixing bowl (10) and/or the applicator (20).

12. A process for transferring bone cement with an apparatus according to any one of claims 2 to 11, characterized in that liquid monomer and polymer powder are contacted and mixed in the mixing bowl (10) and the bone cement mixture is subsequently extruded through the opening in the applicator (20) by means of compression.

## Revendications

1. Dispositif de transvasement d'un mélange visqueux de substances constitué d'au moins deux composants, comprenant
a) un premier récipient (10) fermé à une extrémité,
b) un deuxième récipient (20), dont la section transversale est plus petite que celle du premier récipient (10), dont l'extrémité tournée vers le premier récipient présente une ouverture, dont l'extrémité opposée au premier récipient est perméable aux gaz et qui est déplaçable dans le premier récipient (10) et par rapport à ce dernier, et
c) un dispositif d'étanchéité (30) agencé à la périphérie extérieure du deuxième récipient (20) à proximité de son extrémité ouverte, qui, lors d'un mouvement relatif entre les deux récipients effectué par un enfoncement du deuxième récipient (20) avec son extrémité en avant dans le premier récipient (10), étanchéifie pour le mélange de substances et ses composants l'espace intermédiaire compris entre la périphérie intérieure du premier récipient (10) et la périphérie extérieure du deuxième récipient (20) et qui présente une ouverture libre pour le passage du mélange visqueux de substances en vue de l'extrusion du mélange de substances du premier récipient (10) dans le deuxième récipient (20).

2. Dispositif suivant la revendication 1, caractérisé en ce que le mélange visqueux de substances est du ciment d'os, en ce que le premier récipient (10) est un godet de mélange ayant un fond (12) et en ce que le deuxième récipient (20) est un récipient d'application.

3. Dispositif de transvasement de ciment d'os, comprenant
a) un godet de mélange (10) présentant un fond (12),
b) un récipient d'application (20), dont la section transversale est plus petite que celle du godet de mélange (10) et qui est déplaçable dans le godet de mélange (10) et par rapport à ce dernier, l'extrémité du récipient d'application (20) qui est tournée vers le fond (12) du godet de mélange (10) présentant une ouverture pour le passage du ciment d'os et l'extrémité du récipient d'application (20), qui est à l'opposé du fond (12) du godet de mélange (10), étant perméable aux gaz, et
c) un dispositif d'étanchéité (30) couplable au récipient d'application (20) pour réaliser une étanchéité vis-à-vis du ciment entre la périphérie extérieure du récipient d'application (20) et la périphérie intérieure du godet de mélange (10).

4. Dispositif suivant l'une des revendications 2 et 3, caractérisé par un couvercle (50) prévu pour fermer de manière étanche au vide le godet de mélange (10) et présentant une tubulure (58) pour le raccordement d'un conduit à vide.

5. Dispositif suivant la revendication 4, caractérisé en ce que le couvercle (50) présente un bord solide (52) et une pièce interne (60) constituée en une matière flexible et présentant un passage (62) pour une barre ronde (64).

6. Dispositif suivant l'une des revendications 2 à 5, caractérisé en ce que le godet de mélange (10) est cylindrique et en ce que son fond est réalisé sous une forme circulaire ou concave à l'intérieur.

7. Dispositif suivant l'une des revendications 1 à 6, caractérisé en ce que le dispositif d'étanchéité (30) présente au moins une soupape pour le passage de gaz.

8. Dispositif suivant l'une des revendications 1 à 7, caractérisé en ce que le dispositif d'étanchéité (30) présente sur sa périphérie extérieure un corps cylindrique (32) comportant plusieurs lamelles flexibles (34) qui sont en appui sur la périphérie intérieure du godet de mélange (10).

9. Dispositif suivant la revendication 8, caractérisé en ce qu'au moins la lamelle avant, faisant face au fond du godet de mélange, est concave.

10. Dispositif suivant l'une des revendications 2 à 9, caractérisé par une embouchure (24) couplable ou reliée à une extrémité du récipient d'application (20).

11. Dispositif suivant l'une des revendications 2 à 10, caractérisé par un dispositif de mise sous vide du godet de mélange (10) et/ou du récipient d'application (20).

12. Procédé de transvasement de ciment d'os à l'aide d'un dispositif suivant l'une des revendications 2 à 11, caractérisé en ce que du monomère fluide et de la poudre de polymère sont réunis et mélangés dans le godet de mélange (10) et en ce que le mélange de ciment d'os est ensuite extrudé par compression à travers l'ouverture dans le récipient d'application (20).
